Europäisches Patentamt

European Patent Office

Office européen des brevets

(11) Publication number: 0 453 014 A1

# EUROPEAN PATENT APPLICATION

(21) Application number: 91200725.9

(51) Int. Cl.5: A61B 8/08

(22) Date of filing: 28.03.91

(30) Priority: 20.04.90 NL 9000939

(43) Date of publication of application:
23.10.91 Bulletin 91/43

(84) Designated Contracting States:
AT BE CH DE DK ES FR GB GR IT LI LU NL SE

(71) Applicant: Technische Universiteit Delft
P.O.Box 5046
NL-2600 GA Delft(NL)

(72) Inventor: Berkhout, Augustinus Johannes
Résidence 'Witte Brug', Nw. Parklaan 30
NL-2597 LD Den Haag(NL)
Inventor: Van Hoorn, Willem Allard
Van Bossestraat 30
NL-2613 CR Delft(NL)

(74) Representative: Flamman, Han
LIOC Patents and Licensing P.O. Box 85096
NL-3508 AB Utrecht(NL)

(54) Scanner for ultrasonic scanning of surfaces.

(57) Scanner for scanning surfaces with ultrasonic signals, in particular surfaces of parts of the body, provided with a scanner head (5) for the transmission of ultrasonic signals and the reception of the echoes hereof. The time passed between the emission of the signal and the reception of the echoes thereof, is a measure for the penetration depth into the surface. The scanner head (5) fits in a holder (6), which is provided with a foil window (7) to be fixed against the surface to be scanned, letting the ultrasonic signals pass through. It may be filled entirely with a liquid. The scanner has been fitted with means with which the scanner head may be moved parallel to the surface to be scanned, the ultrasonic signals hitting this surface under a fixed angle. Various constructions for moving the scanner head are included.

Fig. 1

The invention relates to a scanner for scanning surfaces with ultrasonic signals, in particular surfaces of parts of the body, provided with a scanner head for the transmission of ultrasonic signals and the reception of the echoes hereof, at which the time passed between the emission of the signal and the reception of the echoes thereof, is a measure for the penetration depth into the surface, which scanner head has been fitted into a liquid-proof holder, which holder is provided with a window to be fixed against the surface to be scanned for the ultrasonic signals and may be entirely filled with a liquid.

Such a construction is known, at which the scanner head may be turned around an axis parallel to the surface to be scanned. The disadvantage is that the distance from the scanner head until the surface to be scanned varies strongly during scanning, just as the angle under which the ultrasonic signals fall on the surface.

This disadvantage according to a characteristic of the scanner according to the invention is avoided by fitting the scanner with means with which the scanner head may be moved parallel to the surface to be scanned and the ultrasonic signals hitting this surface under a fixed angle.

According to a further characteristic of a scanner according to the invention, the means consist of a guide with which the scanner head may be shifted directly or by means of a screwed spindle.

This makes it possible to scan the structure of materials, for instance the tissue beneath the skin, in an area, for example perpendicular to the surface and to trace possible abnormalities herein, for instance by a tumour.

According to another characteristic of a scanner according to the invention, the scanner head is rotatable around an axis perpendicular to the surface to be scanned and lying outside the axis of the scanner head itself. This enables a circular or in case of a variation of the radius of rotation, a spiral scanning, which makes it amongst others possible to determine the spacial growth of a tumour.

According to again another characteristic of the invention, the scanner head may be rotated around an axis parallel to the direction of motion of the guide. This makes it possible to scan hollow objects, such as an oesophagus, in a circular way with a scanner head that is directed outwards and, in combination with a rectilinear movement according to a helix.

According to another characteristic of a scanner according to the invention, the scanner head, possibly with holder, is connected with this guide by means of an element, mainly stretching into the direction of motion of the guide, preferably resilient the scanner head standing back herefrom and being pressed moveably against the surface by means of a slot in an auxiliary that may serve as holder and closely fitting in a hollow body to be scanned. This is very useful in case of establishing the penetration depth of a tumour, for instance in a cervix and a cervix-mouth respectively.

Another form of construction is characterized by electronic means of indicating location in order to determine the position of the scanner head. These means of indicating location may for instance be of the electromagnetic type with a magnetic tape or of the optical type with glass rulers. The outward signals of the means of indicating location may be used, according to another characteristic of the invention, to produce the ultrasonic signals, whereas a calculator and/or a monitor is present to process the echoes and to make them visible.

All this will now be elucidates further by way of an example by means of the following figure description, in which:

Figure 1 shows a scanner with a guide in a side view;

Figure 2 shows the same scanner in a bottom view;

Figure 3 shows a scan button that may be rotated around an axis perpendicular to the surface;

Figure 4 shows a scanning head to scan hollow bodies and

Figure 5 shows an apparatus for cervix examination.

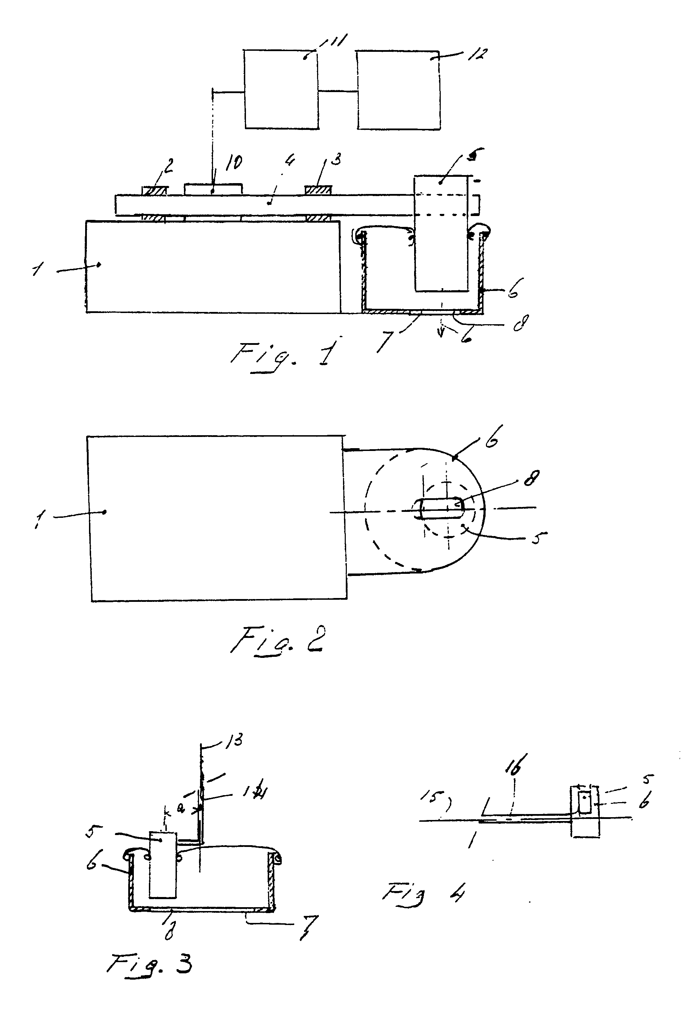

In Figure 1 and 2, there is on house 1 a guide consisting of two parts 2 and 3, through which a rod 4 has been slidable adjusted. On rod 4, a scanner head 5 has been adjusted, which radiates ultrasonic signals 6 and receives the echoes hereof. The scanning head 5 is located in a holder 6 filled with a liquid, which is provided at the bottom with a slot 8 closed by a foil 7 to let the ultrasonic signals pass through. At the top, the holder 6 is liquid-proof closed off by a flexible membrane 9, through which sticks the scanning head 5. As liquid, water may be used. The rod 4 is provided with electronic means of indicating location, which may consist of an electromagnetic strip or of an optical ruler, with a measuring head 10, the outward signals of which may serve to (have) create(d) on certain places or series of places the ultrasonic signal for the scanning head 5, after which the echoes may be processed in a calculator 11 and made visible on a monitor 12.

The rod 4 may be moved by means of a screwed spindle or by moving it by hand against the pressure of a spring and then to have this spring faintly relax, by which a regular scanning movement will take place. The scanning head may have the ultrasonic signals pass perpendicular through the foil 7, but it may be necessary, in connection with undesired echoes, for instance of the foil 7, to send the signals under a different angle through the foil 7. It may also be possible to use two or more scanning heads, possibly in a row.

In Figure 3 it is indicated how the scanning head may be rotatably executed around an axis perpendicular to the surface to be scanned. The scanning head 5 is rotatable around the axis 13 fitted on a rod 14 in the holder 6. It will be evident that the aperture 8 must be circular just as the foil 7.

By making the distance 'a' adjustable, the diameter of the turning circle may be adjusted. By changing the distance 'a' during the turning, it will be possible to scan the surface according to a spiral.

Figure 4 shows how the scanning head 5 has been fitted rotatably around the axis 15 on the rod 6 in the holder 6, at which the axis 15 is parallel to the direction of motion of the guide. In this way, it will be possible to circularly scan hollow objects, such as an oesophagus, with the outward directed scanning head 5. By moving the scanning head simultaneously with the guide along the axis 15, a helical scanning may be obtained. By for instance making the rod 16 elastic, one may achieve that the scanning head always brushes against the side of the object to be scanned.

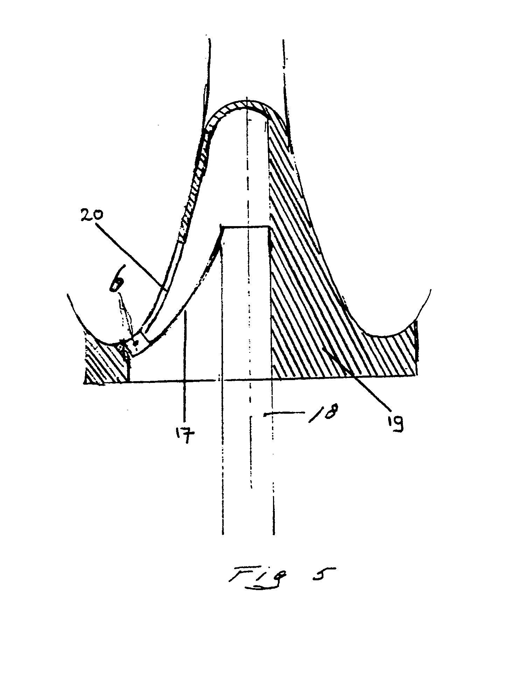

In Figure 5 it is indicated how the holder 6, in which a scanning head is located, is connected via a spring 7 with a rod 18 adjusted to the guide of the scanner, which may move within an auxiliary 19. This auxiliary is provided with a slot 20, through which sticks the holder 6 and closely fits to the body to be scanned, in this case a cervix or cervix-mouth respectively. By moving the rod 18 in the longitudinal direction, the holder 6 will slide through the slot and will thus scan the end of the cervix or cervix-mouth respectively. It will also be possible to seal off the slot on the outside with a foil and to use the auxiliary as a holder, at which the bottom of the auxiliary fits liquid-proof to the rod 18. This is applied in research for cancer of the cervix or cervix-mouth respectively.

## Claims

1. Scanner for scanning surfaces with ultrasonic signals, in particular surfaces of parts of the body, provided with a scanner head for the transmission of ultrasonic signals and the reception of the echoes hereof, at which the time passed between the emission of the signal and the reception of the echoes thereof, is a measure for the penetration depth into the surface, which scanner head has been fitted in a holder, which holder is provided with a window to be fixed against the surface to be scanned, letting the ultrasonic signals pass through and may be entirely filled with a liquid, characterized in that the scanner has been fitted with means with which the scanner head may be moved parallel to the surface to be scanned, the ultrasonic signals hitting this surface under a fixed angle.

2. Scanner according to claim 1, characterized in that the means consist of a guide with which the scanner head may be shifted directly of by means of a screwed spindle.

3. Scanner according to claim 1 or 2, characterized in that the scanner head is rotatable around an axis perpendicular to the surface to be scanned and lying outside the axis of the scanner head itself.

4. Scanner according to claim 1 or 2, characterized in that the scanner head may be turned around an axis parallel to the surface to be scanned.

5. Scanner according to claim 1 or 2, characterized in that the scanner head, possibly with a holder, is connected with this guide, by means of an element, mainly stretching into the direction of motion, preferably resilient, the scanner head standing back herefrom and being pressed moveably against the surface by means of a slot in an auxiliary that may serve as holder and closely fitting in a hollow body to be scanned.

6. Scanner according to claim 1 - 5, characterized in that electronic means of indicating location are present in order to determine the position of the scanner head.

7. Scanner according to claim 6, characterized in that the outwards signals of the means of indicating location may be used to produce the ultrasonic signals, whereas a calculator and/or a monitor is present to process the echoes and to make them visible.

Fig. 1

Fig. 2

Fig. 3

Fig. 4

Fig 5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,A | ELECTRONICS. vol. 28, no. 3, March 1955, New York (US). pages 174 - 180; WILD et al.: "Ultrasonic ranging speeds cancer diagnosis." <br> * page 174, line 69 - page 176, line 4; figure 1 * * page 180, lines 32 - 40; figure 12 * <br> – – – | 1,2,6,7 | A 61 B 8/08 |
| X,A | EP-A-0 320 444 (LABORATORY EQUIPMENT, CORP.) <br> * column 3, line 57 - column 7, line 56; figures 1-5 * <br> – – – | 1,2,7 | |
| X,A | IRE CONVENTION RECORD. vol. 3, no. 9, March 1955, pages 68 - 74; REID et al.: "Ultrasonic echo-ranging for tissue diagnostic studies." <br> * page 69, line 1 - page 71, line 57; figure 4 * <br> – – – | 1,2,4,6,7 | |
| X,A | DE-A-3 227 624 (SIEMENS AG) <br> * page 8, line 11 - page 13, line 30; figures * <br> – – – | 1,2-4,6,7 | |
| A | DE-A-2 305 501 (SIEMENS AG) <br> * page 4, line 10 - page 5, line 17; figures 1, 2 * <br> – – – – – | 1,2,4 | |
| | | | **TECHNICAL FIELDS SEARCHED (Int. Cl.5)** |
| | | | A 61 B <br> G 10 K <br> G 01 N |

The present search report has been drawn up for all claims

| Place of search | Date of completion of search | Examiner |
|---|---|---|
| The Hague | 30 July 91 | CHEN A.H. |